# EUROPEAN PATENT APPLICATION

(11) **EP 1 980 247 A1**
(43) Date of publication of application: **15.10.2008**
(21) Application number: 07707639.6
(22) Date of filing: 29.01.2007
(51) Int. Cl.: A61K 31/198, A23L 1/305, A61K 8/44, A61P 3/04, A61P 3/06, A61P 3/10, A61P 5/10, A61P 17/00, A61P 19/00, A61P 21/00, A61P 39/00, A61Q 19/00, A61Q 19/08

(54) **GROWTH HORMONE SECRETION REGULATOR**

(30) Priority: 30.01.2006 JP 2006020851
(71) Applicant: Ajinomoto Co., Inc., Tokyo 104-8315 (JP)
(72) Inventor: ONO, Kaori, Kanagawa 210-8681 (JP); YONEZAWA, Tomohiro, Kanagawa 210-8681 (JP); MORI, Masato, Kanagawa 210-8681 (JP)
(74) Representative: Nicholls, Kathryn Margaret
(86) International application number: PCT/JP2007/051408
(87) International publication number: WO 2007/086565

(57) **Abstract**

The present invention provides a growth hormone secretion regulator or an anti-aging agent comprising aspartic acid and valine as active ingredients. The present invention also provides a pharmaceutical, food, nutritional supplement (supplement), or feeding stuff comprising the growth hormone secretion regulator or anti-aging agent.

## Description

### Technical Field

The present invention relates to a growth hormone secretion regulator and anti-aging agent, and a pharmaceutical, food, nutritional supplement (supplement), or feeding stuff, which contain the growth hormone secretion regulator or anti-aging agent.

### Background Art

A growth hormone is a 22-kDa peptide hormone secreted from the pituitary gland. The growth hormone can provide various effects such as promotion of bone formation, increase in muscle mass, reduction in the amount of fat, thickening of skin, improvement of fertility, improvement of quality of life (QOL), or the like. Growth hormone is known to be secrete in a pulse-like manner, and it is elucidate that expression of the effects is largely affected by a secretion pattern of a growth hormone secreted in a pulse-like manner. Moreover, the amount of a growth hormone secreted is decreased with aging, and the secretion pattern is changed. As a result, the above-mentioned effects are reduced, resulting in causing symptoms of aging (Non-patent Document 1).

In order to improve various symptoms caused by a decrease in secretion of a growth hormone, there have been developed a method of administering a growth hormone, a substance to promote secretion of a growth hormone, and the like, which have been used for patients with growth hormone deficiency and elderly people (Patent Documents 1 and 2). However, these conventional arts are technologies developed in disregard of a pulsed secretion pattern of a growth hormone. Conventional administration of a growth hormone may change the pattern of the concentration process of a growth hormone in blood, resulting in the induction of not only the above-mentioned beneficial effects but also adverse effects such as insulin resistance (Non-patent Document 1).

The pulsed secretion of a growth hormone is considered to provide an excellent effect of suppressing obesity and increasing bone mass and an effect of suppressing induction of insulin resistance compared with a continuous secretion (Non-patent Documents 2 and 3).
Patent Document 1: US 5,654,278
Patent Document 2: WO 00/64283 A1
Non-patent Document 1: Corpas E, Harman SM, Blackman MR. Human growth hormone and human aging. Endocrine Reviews. 14 (1):20-39, 1993.
Non-patent Document 2: Ikeda A, Chang KT, Matsumoto Y, Furuhata Y, Nishihara M, Sasaki F, Takahashi M. Obesity and insulin resistance in human growth hormone transgenic rats. Endocrinology 139: 3057-63, 1998.
Non-patent Document 3: Jansson JO, Albertsson-Wikland K, Eden S, Thorngren KG, Isaksson O. Circumstantial evidence for a role of the secretory pattern of growth hormone in control of body growth. Acta Endocrinologica 99:24-30, 1982.

### Disclosure of the Invention

### Problems to be solved by the Invention

An object of the present invention is to provide an agent for preventing or ameliorating deterioration of health conditions due to an age-related reduction in growth hormone secretion such as a decrease in the secreted amount of a growth hormone and a change in the secretion pattern. Specifically, an object of the present invention is to provide: a growth hormone secretion regulator which regulates the secreted amount of a growth hormone that is reduced with aging and changes in the secretion pattern; an anti-aging agent which is effective for improvement of insulin resistance, anti-obesity, promotion of lipolysis, increase in muscle mass, increase in bone density, and improvement of skin roughness; or a pharmaceutical, food, nutritional supplement (supplement), or feeding stuff, which is effective for anti-aging.

### Means for solving the Problems

The inventors of the present invention have extensively carried out research with a view to resolving the above-mentioned problems and found out that a composition including aspartic acid and valine could regulate the secreted amount of a growth hormone that is reduced with aging, and secretion pattern that is changed with aging, thus completing the present invention. That is, the summary of the present invention is as follows:
(1) a growth hormone secretion regulator including aspartic acid and valine as active ingredients;
(2) a growth hormone secretion regulator according to Item (1), in which the growth hormone secretion regulator increases an amount of a growth hormone secreted in a pulse-like manner;
(3) a growth hormone secretion regulator according to Item (1), in which the growth hormone secretion regulator enhances pulsed secretion of the growth hormone;
(4) an anti-aging agent including aspartic acid and valine as active ingredients;
(5) an anti-aging agent according to Claim 4, wherein the anti-aging comprises preventing or ameliorating aging selected from the group consisting of anti-hyperglycemia, anti-obesity, promotion of lipolysis, increase in muscle mass, increase in bone density, and improvement of skin roughness;
(6) a growth hormone secretion regulator or anti-aging agent according to any one of Items (1) to (5), in which a mass ratio of aspartic acid to valine is 1 : 2 to 6 : 1;
(7) a pharmaceutical, food, nutritional supplement, or feeding stuff including a growth hormone secretion regulator or anti-aging agent according to any one of Items (1) to (6);
(8) a use of aspartic acid and valine as growth hormone secretion regulators;
(9) a use of aspartic acid and valine as anti-aging agents;
(10) a method of regulating secretion of a growth hormone, which includes administering aspartic acid and valine; and
(11) a method of preventing or ameliorating aging, which includes administering aspartic acid and valine.

### Effect of the Invention

The present invention provides an agent for preventing or ameliorating deterioration of health conditions due to an age-related reduction in growth hormone secretion such as a decrease in the secreted amount of a growth hormone and a change in the secretion pattern. Specifically, the present invention provides: a growth hormone secretion regulator which regulates the secreted amount of a growth hormone that is reduced with aging and secretion pattern that is changed with aging; an anti-aging agent which is effective for improvement of insulin resistance, anti-obesity, promotion of lipolysis, increase in muscle mass, increase in bone density, and improvement of skin roughness; or a pharmaceutical, food, nutritional supplement (supplement), or feeding stuff, which is effective for anti-aging.

### Brief Description of the Drawings

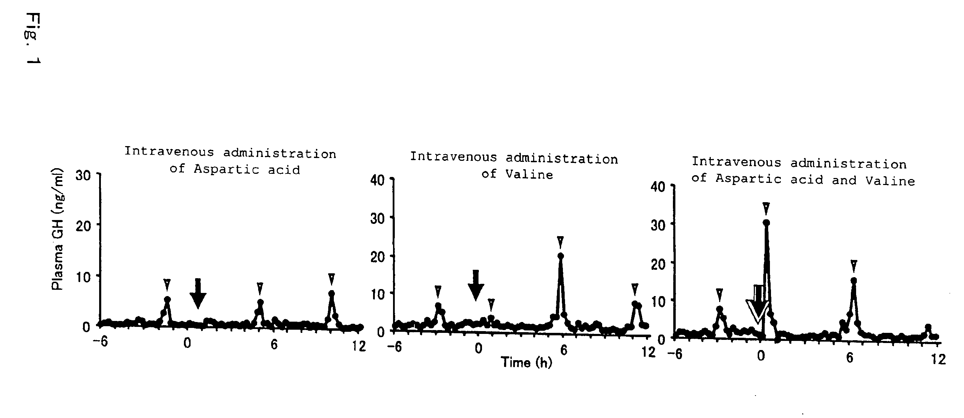

Figs. 1 are graphs showing changes (typical cases) in growth hormone secretion caused by intravenous administration of amino acid solutions. The graphs show the concentrations of the growth hormone in blood during a period between 6 hours before and 12 hours after administration of amino acid solutions. The arrows show times when each amino acid solution was administered. The inverted triangles show peaks of pulsed secretion. Intravenous administration of aspartic acid and valine was found to induce immediate pulsed release of the GH and enhancement of pulse at next time.

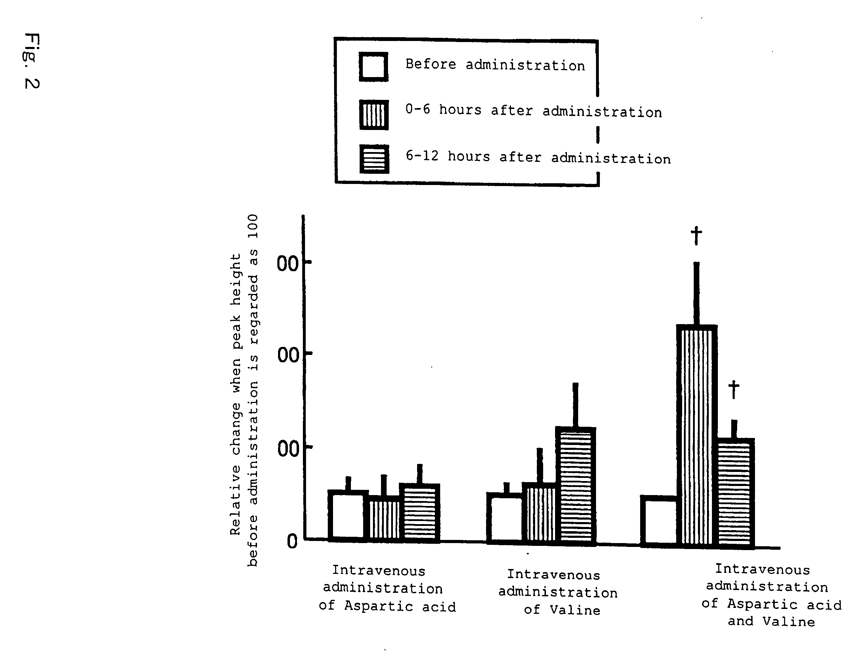

Fig. 2 is a graph showing relative changes (n = 3) in peak heights of a growth hormone secreted in a pulse-like manner by intravenous administration of amino acid solutions. The graph shows mean values + SEMs of the highest peaks in pulsed secretion for a period of 6 hours before administration of the amino acid solution (blank bar), a period of 0 to 6 hours after administration (vertical-striped bar), and a period of 6 to 12 hours after administration (horizontal-striped bar) (t: P < 0.1 vs before administration). The graph shows that mixing of aspartic acid and valine can provide a synergistic effect.

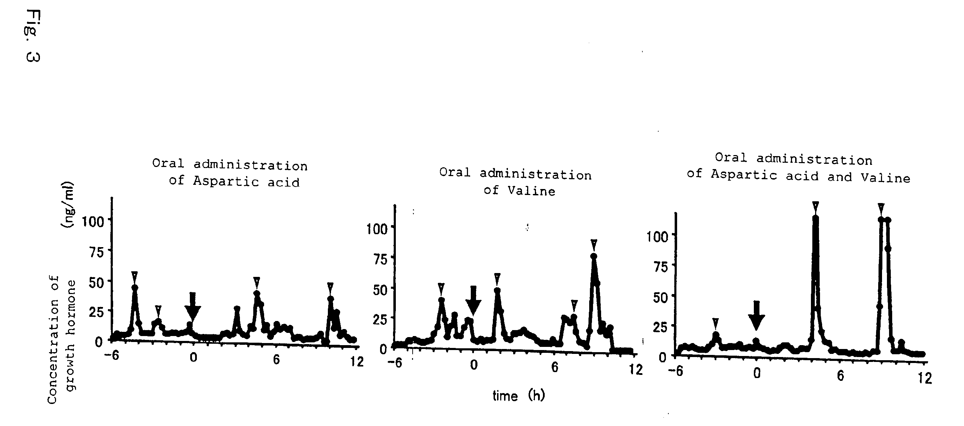

Figs. 3 are graphs showing changes (typical cases) in growth hormone secretion caused by oral administration of amino acid solutions. The graphs show the concentrations of the growth hormone in blood during a period between 6 hours before and 12 hours after administration of amino acid solutions. The arrows show times when each amino acid solution was administered. The inverted triangles show peaks of pulsed secretion. Oral administration of aspartic acid and valine was found to induce increases in the peak heights of the pulsed secretion at next time and the time after the next time.

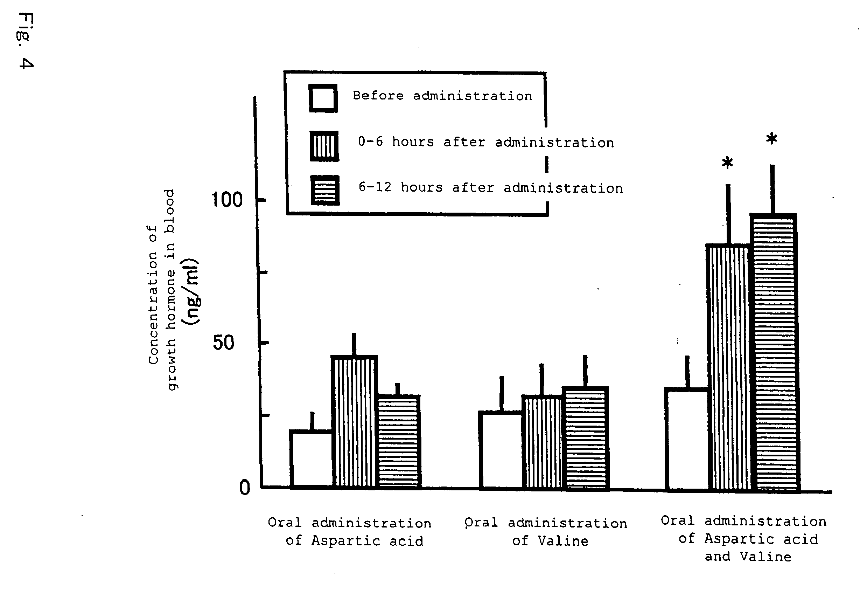

Fig. 4 is a graph showing changes (n = 3) in peak heights of a growth hormone secreted in a pulse-like manner by oral administration of amino acid solutions. The graph shows mean values + SEMs of the highest peaks in pulsed secretion for a period of 6 hours before administration of the amino acid solution (blank bar), a period of 0 to 6 hours after administration (vertical-striped bar), and a period of 6 to 12 hours after administration (horizontal-striped bar) (*: P < 0.1 vs before administration). The graph shows that mixing of aspartic acid and valine can provide a synergistic effect.

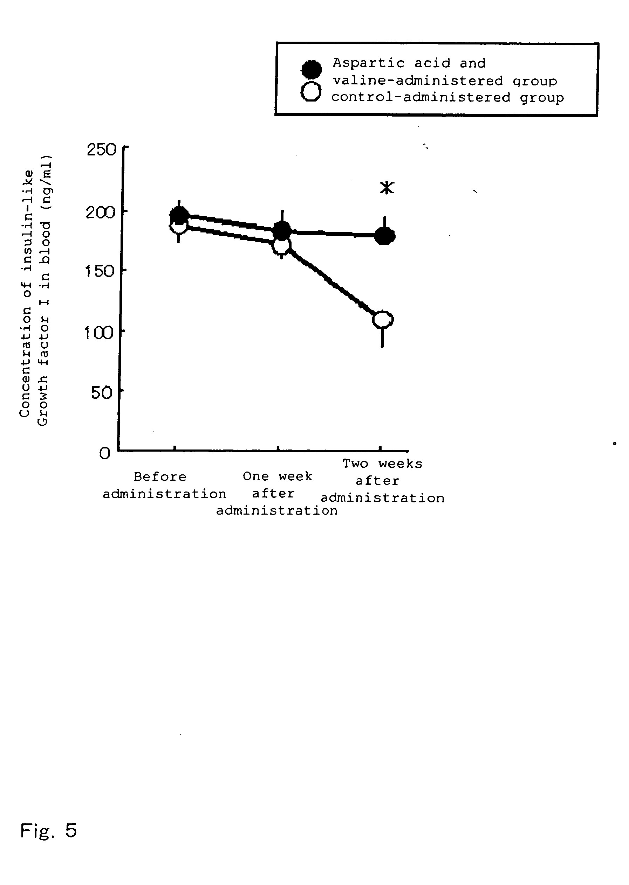

Fig. 5 is a graph showing changes in the concentrations of insulin-like growth factor I in blood caused by daily oral administration of a mixed solution of aspartic acid and valine. The graph shows the concentrations of insulin-like growth factor I in blood of db/db mice before administration, one week after administration, and two weeks after administration. Only a solvent was administered orally to the control group every day. As a result, the two-week oral administration of the mixed solution of aspartic acid and valine was found to significantly increase the concentrations of insulin-like growth factor I in blood (*: P<0.05, vs control-administered group).

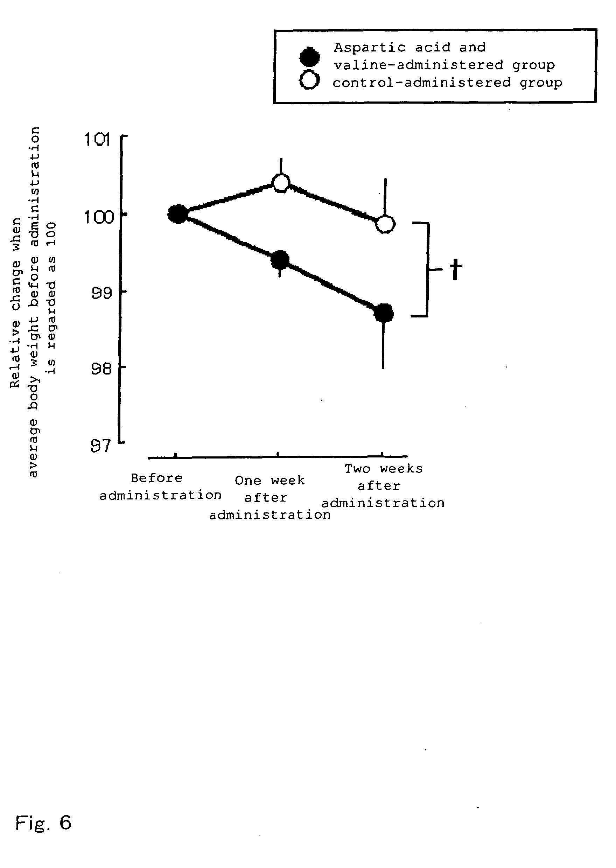

Fig. 6 is a graph showing relative changes in average weekly body weights caused by daily oral administration of a mixed solution of aspartic acid and valine. Only a solvent was administered orally to the control group every day. The relative changes in average body weights of db/db mice before administration, one week after administration, and two weeks after administration when body weights before administration are regarded as 100. As a result, the two-week oral administration of the mixed solution of aspartic acid and valine was found to significantly decrease the body weights (t: P < 0.1, aspartic acid and valine-administered group vs control-administered group, two-way analysis of variation).

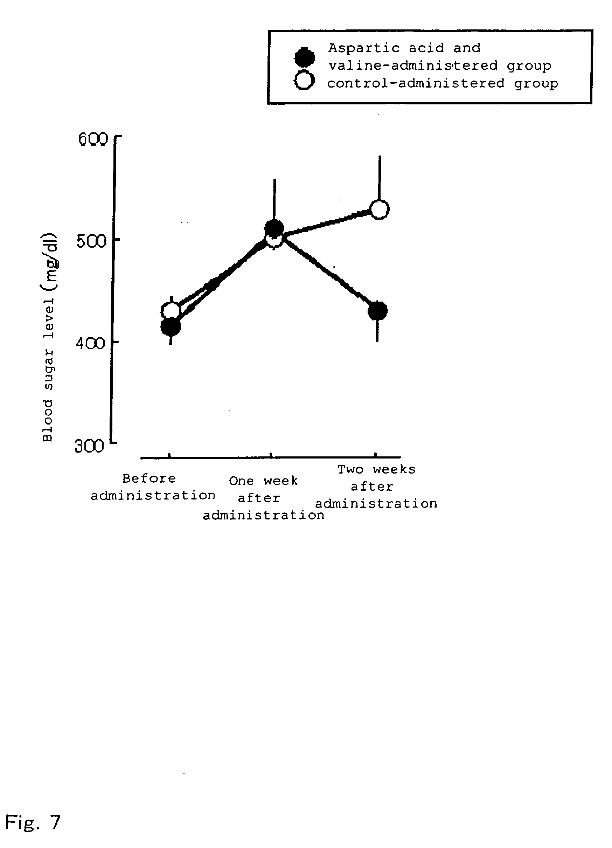

Fig. 7 is a graph showing changes in blood sugar levels in full feeding caused by daily oral administration of a mixed solution of aspartic acid and valine. Only a solvent was administered orally to the control group every day. The graph shows blood sugar levels in full feeding of db/db mice before administration, one week after administration, and two weeks after administration. There is no statistically significant difference, but the two-week oral administration of the mixed solution of aspartic acid and valine decreased the blood sugar levels in full feeding.

### Best Mode for carrying out the Invention

Hereinafter, the present invention will be described in detail.

### <1> Growth hormone secretion regulator of the present invention

The growth hormone secretion regulator of the present invention (hereinafter, also referred to as "GH secretion regulator of the present invention") contains aspartic acid and valine as active ingredients. In the description of the present invention, the term "amino acid" refers to an L-type amino acid unless otherwise specified.

Note that the term "growth hormone secretion regulator" as used herein refers to: an agent having the effect of increasing the amount of a growth hormone when secreted in a pulse-like manner without changing the secretion pattern of the growth hormone secreted in a pulse-like manner at a certain frequency; an agent having the effect of enhancing the pulsed secretion of a growth hormone; or an agent having both of the effects. The increase in the amount of secreted growth hormone can be conf irmed by determining an increase in the height or the like of a secretion peak of the growth hormone secreted in a pulse-like manner. Moreover, the growth hormone secretion regulator has the effect of inducing the pulsed secretion of a growth hormone, and therefore it is possible to regulate the secretion pattern of the growth hormone by an administration method so as to secrete the growth hormone in a pulse-like manner, that is, it is possible to regulate the secretion pattern of the growth hormone by regular administration so as to secrete the growth hormone at regular intervals in a pulse-like manner.

In the present invention, the term "secretion in a pulse-like manner (sometimes referred as "pulsed secretion")" refers to secretion to reach a sufficient high level from a basal level, which occurs once every 3 to 6 hours for about 1 hour, and it is identified as a pulse by the PULSAR algorithm described in American Journal Physiology 243:E310-318, 1982.
The PULSAR algorithm is a calculational method for identifying a pulse in time-series data, where a point beyond the average of time-series data is compared with an error range in an assay to identify the point as a pulse if the value is beyond the threshold level.

As shown in examples, aspartic acid and valine to be used in the present invention can be used as growth hormone secretion regulators because they can increase the height of a peak of a growth hormone in pulsed secretion that is caused within about 12 hours after ingestion of aspartic acid and valine, can induce pulsed secretion even when pulsed secretion of a growth hormone is originally not caused, and can significantly increase the height of the secretion peak compared with a case where no amino acid is added.
It has been reported that the mechanism of regulation of growth hormone secretion by amino acid administration is provided by promotion of secretion of a growth hormone releasing hormone, suppression of somatostatin secretion, and the like, but another report suggests that the mechanism may be provided by a change in metabolism.

In the growth hormone secretion regulator of the present invention, the mixing ratio of aspartic acid to valine to be used in the present invention is not particularly limited as long as it can provide the above-mentioned growth hormone secretion regulating function, and the mass ratio of aspartic acid to valine is preferably 1 : 2 to 6 : 1, and more preferably 2 : 1 to 4 : 1.

The above-mentioned aspartic acid and valine may be commercially available products.
In addition, aspartic acid to be used in the present invention may be in a salt form. In the case where aspartic acid to be used in the present invention is in a salt form, the salt may be a pharmacologically acceptable salt, and examples thereof include salts of an acidic group such as a carboxyl group in the formula with: ammonium; an alkaline metal such as sodium or potassium; an alkaline earth metal such as calcium or magnesium; aluminum; zinc; an organic amine such as triethylamine, ethanolamine, morpholine, pyrrolidine, piperidine, piperazine, or dicyclohexylamine; and a basic amino acid such as arginine or lysine.

In measurement of the growth hormone secretion regulating function,
1) a mammal to be used as an experimental animal is preferably a shiba-goat (*capra hircus*), rat, etc. because so many endocrinological study report that pulsed pattern of a growth hormone secretion can be easily observed. The animal is particularly preferably a mature castrated male and female shiba-goat.
2) The experiment animal is preferably domesticated for about one week before administration of a test substance to the animal so that the animal becomes comfortable with human and has no or little stress.
3) In order to orally administer a test substance to an experimental animal, an aqueous solution of the test substance is administered to the stomach using a feeding needle, or the animal is allowed to drink the aqueous solution. On the other hand, in order to parenterally administer a test substance to an experimental animal, a test substance is preferably administered intravenously via a cannula placed in the vein (such as jugular vein) for collecting blood.
4) In a method of collecting blood from an experimental animal and a method of measuring the amount of a growth hormone,
in the case where blood is collected from the animal at certain intervals, a conventional blood collection method may be employed. For example, a cannula is preliminarily placed in the vein (such as jugular vein) of an experimental animal, and blood is collected every 15 minutes in a predetermined amount (about 0.2 to 5.0 ml). After blood collection, plasma is separated from the resultant blood by centrifugation or the like and is used to measure a growth hormone by a conventional method such as radioimmunoassay or ELISA in which an anti-growth hormone antibody is used.

### <2> Anti-aging agent of the present invention

The anti-aging agent of the present invention contains the above-mentioned growth hormone secretion regulator as an active ingredient. Moreover, the anti-aging agent of the present invention contains aspartic acid and valine as active ingredients.

Note that, the term "anti-aging" as used herein refers to prevention and improvement of impaired physiological functions caused by a decrease in the secreted amount of a growth hormone, a change in the secretion pattern, and the like owing to aging, nutritional status, diseases, etc. Specific examples thereof include, but are not limited to, anti-hyperglycemia due to insulin resistance, anti-obesity, promotion of lipolysis, increase in muscle mass, increase in bone density, and improvement of skin roughness.

A growth hormone (GH) has the effect of increasing muscle mass, decomposing fat, and promoting bone formation. The GH is secreted in a pulse-like manner, and the secretion pattern is known to play an important role in expression of GH effects.

For example, it has been found that a rat modified so as not to secrete a GH in a pulse-like manner has increased amount of fat, is more likely to develop diabetes (insulin resistance), and has reduced bone density (Yamonouchi K et al., Experimental Gerontology 2004; 39: 1179-88, Ikeda A at al., Endocrinology 1998; 139: 3057-63).

Meanwhile, in a goat in estrous phase in which pulsed secretion of a GH is enhanced, the concentration of IGF-I in blood, an indicator of muscle enhancement, is higher than that of a goat in luteal phase in which pulsed secretion of a GH is suppressed (Yonezawa T et al., Endocrinology 2005; 146: 2736-46).

Administration of a GH in a pulse-like manner to a rat lacking an endogenous GH is known to provide a significant elongation of the thigh bone and a significant decrease in the epididymal fat weight compared with a case where the GH is administered continuously although the daily total amounts of the GH administered are the same each other (Jannsson JO et al., Acta Endocrinologica 1982; 99: 24-30, Ikeda A et al., Endocrinology 1998; 139: 3057-63).

When the same experiment as above was performed for the human, osteocalcin, an indicator of bone formation, had a tendency to increase (Jaffe CA et al., Am J Physiol Endocrinol Metab 2002; 283: E1008-15).
Those findings reveal that the pulsed secretion of a GH plays an important role in expression of GH effects.

On the other hand, there is a finding of the fact that an insulin resistance is easily developed in the case where only the total amount of a GH administered or the amount of a GH released immediately after bedtime is supplied in a secretion pattern other than the above-mentioned pulsed secretion (Drake WM et al. , Endocrine Reviews 2001; 22: 425-50).

Therefore, the above-mentioned growth hormone secretion regulator which increases the amount of a growth hormone secreted in a pulse-like manner without changing the secretion pattern of the growth hormone secreted in a pulse-like manner at a certain frequency can be used for improvement of insulin resistance, anti-obesity, promotion of lipolysis, increase in muscle mass, increase in bone density, and improvement of skin roughness.
In addition, as demonstrated in examples, aspartic acid and valine which increases the amount of a growth hormone secreted in a pulse-like manner without changing the secretion pattern of the growth hormone secreted in a pulse-like manner at a certain frequency can be used for improvement of insulin resistance, anti-obesity, promotion of lipolysis, increase in muscle mass, increase in bone density, and improvement of skin roughness.

In the anti-aging agent of the present invention, the mixing ratio of aspartic acid to valine to be used in the present invention is not particularly limited as long as it provides the above-mentioned anti-aging effects, and the mass ratio of aspartic acid to valine is preferably 1 : 2 to 6 : 1 and more preferably 2 : 1 to 4 : 1.

### <3> Pharmaceutical, food, nutritional supplement (supplement), or feeding stuff of the present invention

The method of using the above-mentioned growth hormone secretion regulator or anti-aging agent is not particularly limited, and the regulator or agent may be mixed in a pharmaceutical for oral or parenteral use, food for oral use, nutritional supplement (supplement) for oral use, or feeding stuff for oral use.

The above-mentioned growth hormone secretion regulator or anti-aging agent may be used in combination with other various additives (in the case where it is used as a pharmaceutical, a nontoxic carrier for medical use) or the like as a pharmaceutical for oral or parenteral use, food for oral use, nutritional supplement (supplement) for oral use, or feeding stuff for oral use.

The above-mentioned growth hormone secretion regulator or anti-aging agent may further be used as a pharmaceutical for oral or parenteral use, food for oral use, nutritional supplement (supplement) for oral use, or feeding stuff for oral use, which contains an active ingredient thereof in combination with any additive (in the case where it is used as a pharmaceutical, a nontoxic carrier for medical use).

In the case where the above-mentioned growth hormone secretion regulator or anti-aging agent is used as a pharmaceutical, it may be administered orally or parenterally by injection or the like. The above-mentioned growth hormone secretion regulator or anti-aging agent, or an active ingredient thereof may be mixed with a solid or liquid nontoxic carrier for medical use suitable for an administration method such as oral administration or injection and administered in the form of a conventional medicinal preparation. Examples of the preparation include: the forms of solid preparations such as tablets, granules, powders, and capsules; the forms of liquid preparations such as solutions, suspensions, and emulsions; and the forms of freeze-dried preparations. Those preparations may be prepared by pharmaceutically usual methods.

Examples of the nontoxic carrier for medical use include glucose, lactose, sucrose, starch, mannitol, dextrin, fatty acid glyceride, polyethyleneglycol, hydroxyethyl starch, ethylene glycol, polyoxyethylene sorbitan fatty acid ester, gelatin, albumin, amino acids, water, and physiological saline. If necessary, a conventional additive such as a stabilizer, wetting agent, emulsifier,binder,or tonicity-adjusting agent may be appropriately added.

The dose of the growth hormone secretion regulator or anti-aging agent of the present invention is not particularly limited as long as it is effective for treatment or prevention, and it may be appropriately adjusted depending on the patient's age, sex, body weight, degree of symptoms, or the like. For example, in the case of oral administration, the total amount of amino acids to be used in the present invention is preferably 1 mg to 500 mg per kg body weight, and more preferably 20 mg to 100 mg per kg body weight per administration. On the other hand, in the case of parenteral administration, in particular, in a case of using injections, the total amount of amino acids to be used in the present invention is preferably 1 mg to 100 mg per kg body weight, and more preferably 5 mg to 20 mg per kg body weight per administration. The number of administration is not particularly limited, and administration may be performed once or several times a day.
In the case of oral administration to a ruminant such as goat, the total amount of amino acids to be used in the present invention is preferably 10 mg to 2 g per kg body weight, and more preferably 100 mg to 500 mg per kg body weight per administration. On the other hand, in the case of parenteral administration, in particular, in a case of using injections, to a ruminant such as goat, the total amount of amino acids to be used in the present invention is preferably 10 mg to 500 mg per kg body weight, more preferably 50 mg to 200 mg per kg body weight per administration.

In the case of a food or nutritional supplement (supplement) including a seasoning, drink, or the like, the above-mentioned additive is not particularly limited as long as it can be added to and mixed in a food including a seasoning, drink, or the like. Examples of the additive include: flavors; sugars; sweeteners; dietary fibers; vitamins; amino acids such as glutamic acid, arginine, lysine, leucine, and isoleucine; nucleic acids such as inosine monophosphate (IMP); inorganic salts such as sodium chloride and calcium; collagen; and water. Of those, preferable additives are amino acids effective for increasing muscle mass, calcium effective for increasing bone mass, collagen effective for improving skin roughness, etc.

The above-mentioned food or nutritional supplement (supplement) may be formed by a conventional method into a form suitable for esculent, such as granule, particle, powder, tablet, capsule, paste, liquid, or the like and used for esculent or may be added to other foods such as seasonings and drinks.
In addition, the above-mentioned food may be a food packed in a container or package that indicates its anti-aging effect.

The used amount of the growth hormone secretion regulator or anti-aging agent of the present invention with respect to the above-mentioned food or nutritional supplement (supplement) is not particularly limited as long as it is effective for providing the above-mentioned effects, and it is appropriately adjusted depending on the intended use. For example, the amount of the growth hormone secretion regulator or anti-aging agent of the present invention per ingestion (or per unit package, for example) is preferably 50 mg to 30 g and more preferably 2 g to 6 g, which is the total amount of amino acids to be used in the present invention.

In the case of a feeding stuff, the above-mentioned additive is not particularly limited as long as it can be added to and mixed in a feeding stuff. Examples of the additive include: grains such as corn, barley, wheat, rye, sorghum, soybean, and roasted soybean flour; soybean cake and meal; soybean proteins; fatty acids; skimmed milk; fish meals; bone-meal feeds; blood meals; plasma protein; whey; rice bran; wheat bran; saccharides such as sugar; other sweeteners; minerals; vitamins; and salt.
The above-mentioned feeding stuff can be manufactured by a conventional method.

The used amount of the growth hormone secretion regulator or anti-aging agent of the present invention with respect to the above-mentioned feeding stuff is not particularly limited as long as it is effective for providing the above-mentioned effects, and it is appropriately adjusted depending on the intended use. For example, the amount of the growth hormone secretion regulator or anti-aging agent of the present invention per ingestion is preferably 1 mg to 2 g, and more preferably 20 mg to 1,000 mg per kg body weight per administration, which is the total amount of amino acids to be used in the present invention.

### Example 1

### <Evaluation of growth hormone secretion regulating function of amino acids to be used in the present invention (intravenous administration)>

The growth hormone secretion regulating function of amino acids to be used in the present invention was evaluated by investigating changes in growth hormone secretion caused by intravenous administration of amino acid solutions. The details are as follows.

### <Preparation of amino acid solutions>

Special grade aspartic acid (Ajinomoto Co. , Inc.) and valine (Aj inomoto Co., Inc.) were used as L-type amino acid samples. The amino acids were dissolved in purified water or an aqueous solution of 5% carboxymethylcellulose and administered as aqueous solutions (pH 7.0 to 8.0).

### <Evaluation of growth hormone secretion regulating function>

Two- to four-year-old mature castrated male and female shiba-goats (25 to 35 kg), purchased from the farm of Tokyo University, were domesticated for about one week. Aspartic acid (0.75 mmol/kg), valine (1.0 mmol/kg), and a mixture thereof, prepared in the same way as above, were administered intravenously via a cannula placed in the jugular vein, and blood was collected continuously during a period between 6 hours before and 12 hours after administration every 15 minutes via the cannula placed in the jugular vein, followed by investigation of changes in growth hormone secretion (n = 3). The concentrations of the growth hormone in blood were measured by radioimmunoassay based on a double antibody method.
The growth hormone and antibody used in the radioimmunoassay were purchased from National institute of Diabetes and Digestive and Kidney Diseases. The other procedures were performed based on a reference (Hashizume T et al., Animal Science Technology 62: 343-350). The minimum measured concentration was 1 ng/ml, while the maximum measured concentration was 512 ng/ml. The intra-assay variation and inter-assay variation were 11.6% and 12.3%, respectively.
The results are shown in Figs. 1 and 2. As shown in Figs. 1 and 2, in the cases of the single administration groups of aspartic acid and valine, there were no changes in the secretion patterns of the growth hormone secreted in a pulse-like manner at a certain frequency and the amount of the growth hormone secreted in a pulse-like manner. On the other hand, in the case where the mixture of aspartic acid and valine was administered, the pulsed secretion of the growth hormone was promoted immediately after administration while the height of a peak in the next pulsed secretion that occurred several hours later was increased. The results reveal that increases in the concentrations of aspartic acid and valine in blood can induce the pulsed secretion of a growth hormone and can increase the amount of the growth hormone secreted in a pulse-like manner.

### Example 2

### <Evaluation of growth hormone secretion regulating function of amino acids to be used in the present invention (oral administration)>

Two- to four-year-old mature castrated male and female shiba-goats (25 to 35 kg), purchased from the farmof Tokyo University, were domesticated for about one week. Sodium aspartate monohydrate (5 mmol/kg, 861 mg/kg), valine (5 mmol/kg, 586 mg/kg), and a mixture thereof, prepared in the same way as Example 1, were administered orally to the shiba-goats, and blood was collected continuously during a period between 6 hours before and 12 hours after administration every 15 minutes via a cannula placed in the jugular vein, followed by investigation of changes in growth hormone secretion (n = 3). The concentrations of the growth hormone in blood were measured by immunoassay in which europium was used.
The growth hormone and antibody used in the immunoassay were purchased from National institute of Diabetes and Digestive and Kidney Diseases. The other procedures were performed based on a reference (Sugino T et al., Biochemical and biophysical research communications 295: 255-60). The minimum measured concentration was 2 ng/ml, while the maximum measured concentration was 126 ng/ml. The intra-assay variation and inter-assay variation were 11.3% and 8.6%, respectively.
The results are shown in Figs. 3 and 4. As shown in Figs. 3 and 4, in the cases of the single administration groups of aspartic acid and valine, there were no changes in the secretion patterns of the growth hormone secreted in a pulse-like manner at a certain frequency and the amount of the growth hormone secreted in a pulse-like manner. On the other hand, in the case where the mixture of aspartic acid and valine was administered, the pulsed secretion of the growth hormone immediately after administration, as observed in the intravenous administration, was not detected, while the heights of peaks that occurred at a certain frequency in the pulsed secretion at next time and the secretion at the time after the next time were increased. The results reveal that oral administration of aspartic acid and valine can increase the amount of the growth hormone when secreted in a pulse-like manner.

### Example 3

### <Effect of long-period oral administration of amino acids to be used in the present invention on body weight and blood sugar level>

Diabetes and hyperglycaemia model animals, db/db mice (8-week-old, 10 mice in total, average body weight 43.9 g) were purchased from Charles River Laboratories, Inc. and were domesticated for about one week before an experiment. A mixture obtained by blending sodium aspartate monohydrate and valine in a 5% carboxymethylcellulose solution using an agate mortar (sodium aspartate monohydrate 751 mmol/l, and valine 427 mmol/l) was administered orally using a feeding needle to five of the mice once a day on weekdays 10 a.m. to 11 a.m. (10 ml/kg, that is, sodium aspartate monohydrate 1.0 g/kg and valine 0.5 g/kg).
Only a solvent was administered orally to five of the control-administered group in the same manner as above. Administration was continued for two weeks, daily body weight measurement and blood collection immediately once a week before administration were performed to measure blood sugar levels in full feeding and concentrations of insulin-like growth factor I in blood. The blood sugar levels were measured using DRI-CHEM (FUJIFILM Corporation, Japan), and the insulin-like growth factor I was measured by immunoassay in which europium was used.
The samples used in measurement were subjected to an extraction treatment and diluted 50-fold with an assay buffer. The extraction treatment used was the acid-acetone extraction method, which includes: adding the same volume of acetic acid as that of each sample; adding a 4-fold volume of acetone to isolate the supernatant; and evaporating acetone. The above-mentioned hormone and antibody thereof used in the immunoassay of insulin-like growth factor I were purchased from National institute of Diabetes and Digestive and Kidney Diseases. The other procedures were performed in the same way as Example 2. The minimum measured concentration was 15.8 ng/ml, while the maximum measured concentration was 5,000 ng/ml. All the samples were measured in one assay, and the intra-assay variation was 2.28%.
The results are shown in Figs. 5, 6, and 7. As shown in Fig. 5, the two-week administration of aspartic acid and valine increased the concentration of the insulin-like growth factor in blood, an indicator of the amount of secreted growth hormone. Meanwhile, as shown in Figs 6 and 7, in the case of the aspartic acid and valine-administered group, a significant decrease in the body weight and a decrease in the blood sugar level group in full feeding compared to the control-administered group were observed. The results reveal that long-term oral administration of aspartic acid and valine can increase and enhance secretion of a growth hormone and can increase the concentration of an insulin-like growth factor in blood, thereby causing anti-obesity and anti-hyperglycemia effects such as decrease in body weight and decrease in blood sugar level.

### Industrial Applicability

The growth hormone secretion regulator can be used for regulating the secreted amount of a growth hormone that is reduced with aging and changes in the secretion pattern. The anti-aging agent can be used for improvement of insulin resistance, anti-obesity, promotion of lipolysis, increase in muscle mass, increase in bone density, and improvement of skin roughness. In addition, the growth hormone secretion regulator and anti-aging agent can be used as pharmaceuticals, foods, nutritional supplements (supplements), or feeding stuffs containing them. The pharmaceuticals, foods, nutritional supplements (supplements), or feeding stuffs are expected to provide the effects of the present invention in patients with GH deficiency, ruminants, etc.
The present application claims priority from a Japanese patent application (Application Number 2006-020851) filed on January 30, 2006.

## Claims

1. A growth hormone secretion regulator comprising aspartic acid and valine as active ingredients.

2. A growth hormone secretion regulator according to Claim 1, wherein the growth hormone secretion regulator increases an amount of a growth hormone secreted in a pulse-like manner.

3. A growth hormone secretion regulator according to Claim 1, wherein the growth hormone secretion regulator enhances pulsed secretion of the growth hormone.

4. An anti-aging agent comprising aspartic acid and valine as active ingredients.

5. An anti-aging agent according to Claim 4, wherein the anti-aging comprises preventing or ameliorating aging selected from the group consisting of anti-hyperglycemia, anti-obesity, promotion of lipolysis, increase in muscle mass, increase in bone density, and improvement of skin roughness.

6. A growth hormone secretion regulator or anti-aging agent according to any one of Claims 1 to 5, wherein a mass ratio of aspartic acid to valine is 1 : 2 to 6 : 1.

7. A pharmaceutical, food, nutritional supplement, or feeding stuff, comprising the growth hormone secretion regulator or anti-aging agent according to any one of Claims 1 to 6.
